# EUROPÄISCHE PATENTSCHRIFT

(11) **EP 0 743 950 B1**
(45) Veröffentlichungstag und Bekanntmachung des Hinweises auf die Patenterteilung: **12.12.2001**
(21) Anmeldenummer: 95909684.3
(22) Anmeldetag: 08.02.1995
(51) Int. Cl.: C07H 1/08, C07H 21/00, C12N 15/10, C12P 19/34, C12Q 1/68

(54) **VERFAHREN ZUR TRENNUNG VON DOPPELSTRANG/EINZELSTRANGNUKLEINSÄURESTRUKTUREN**
PROCESS FOR SEPARATING DOUBLE-STRANDED/SINGLE-STRANDED NUCLEIC ACID STRUCTURES
PROCEDE DE SEPARATION DE STRUCTURES D'ACIDES NUCLEIQUES A DEUX BRINS ET A UN BRIN

(30) Priorität: 11.02.1994 DE 4404361
(43) Veröffentlichungstag der Anmeldung: 27.11.1996
(60) Teilanmeldung: 01113857.5
(73) Patentinhaber: QIAGEN GmbH, 40724 Hilden (DE)
(72) Erfinder: BASTIAN, Helge, D-30822 Mettmann (DE); GAUCH, Simone, D-42781 Haan (DE); COLPAN, Metin, D-45219 Essen (DE); FEUSER, Petra, D-50933 Köln (DE)
(74) Vertreter: Meyers, Hans-Wilhelm, Dr.Dipl.-Chem.
(86) Internationale Anmeldenummer: EP9500445
(87) Internationale Veröffentlichungsnummer: WO9521849

(56) Entgegenhaltungen:
- EP-A- 0 111 916
- EP-A- 0 325 032
- EP-A- 0 389 063
- EP-A- 0 580 305
- WO-A-91/18116
- WO-A-93/11221
- US-A- 5 075 430
- US-A- 5 155 018
- JOURNAL OF CHROMATOGRAPHY, Bd. 243, 1982 AMSTERDAM (NL), Seiten 301-306, EGLY J.M. ET AL 'Separation of single-stranded from double-stranded nucleic acids using acriflavin-agarose chromatography'
- J. CHROMATOGR. (1985), 324(1), 173-80 CODEN: JOCRAM;ISSN: 0021-9673, SHARIFI, BEHROOZ G. ET AL 'Use of a urea and guanidine-hydrochloric acid-propanol solvent system to purify a growth inhibitory glycopeptide by high-performance liquid chromatography'

## Beschreibung

Gegenstand der vorliegenden Erfindung ist ein Verfahren zur chromatographischen Trennung von Nukleinsäuregemischen in ihre doppel- und einzelsträngigen Nukleinsäureanteile, indem Gesamtnukleinsäuren gleichzeitig an einen mineralischen Träger absorbiert werden und danach die Trennung in doppelsträngige Nukleinsäure und einzelsträngige Nukleinsäure durch fraktionierte Elution erfolgt oder indem doppelsträngige Nukleinsäure oder einzelsträngige Nukleinsäure einer flüssigen Probe selektiv an einen mineralischen Träger absorbiert werden sowie Lösungen und Kit zur Durchführung des erfindungsgemäßen Verfahrens.

Präparation von Nukleinsäuren, sowohl RNA wie auch DNA, hat zunehmend an Bedeutung gewonnen. Dabei werden beispielsweise die biologischen Quellen, aus denen die RNA oder DNA isoliert werden soll, aufgeschlossen, beispielsweise durch mechanische Einflüsse oder chemische Einflüsse, wie Behandlung mit Detergenzien etc. So folgt auf den Zellaufschluß zur Gewinnung der Nukleinsäure üblicherweise eine Cäsiumchlorid-Dichtegradientenzentrifugation oder eine Extraktion mit Phenol. Diese Methoden sind zwar zur Isolation von Nukleinsäuren geeignet, weisen aber Nachteile auf, die ihre Handhabung erschweren. So setzt die Cäsiumchlorid-Dichtegrandientenzentrifugation den Einsatz von zeit- und kostenintensiver Ultrazentrifugation voraus, wohingegen der Umgang mit Phenol aus arbeitsschutzrechtlichen Erwägungen nicht unbedenklich erscheint.

So hat es in der Vergangenheit nicht an Versuchen gefehlt, die Isolation von Nukleinsäuren zu vereinfachen.

Die DE 36 39 949 A1, DE 40 34 036 A1 oder DE 41 39 664 A1 befassen sich z.B. mit Verbesserungen der Nukleinsäurereinigung mittels chromatographischer Methoden unter Vermeidung apparativ aufwendiger Verfahren, wie der Hochdruckflüssigkeitschromatographie (HPLC). Obwohl diese Methoden bereits beispielsweise gegenüber der Ultrazentrifugation oder Phenolextraktion einen Fortschritt darstellen, sind sie technisch relativ aufwendig und arbeitsintensiv. Da zur Fraktionierung oftmals mehrere Reinigungsschritte nacheinander durchgeführt werden müssen, ist die Bearbeitung insbesondere von kleinen Probenmengen z. B. durch Substanzverlust problematisch.

Die EP 0 389 063 A2 betrifft ebenfalls ein Verfahren zur Isolierung von Nukleinsäuren. Dabei wird die Nukleinsäuren enthaltende Quelle in Gegenwart chaotroper Ionen aufgeschlossen und dann mit einem unter diesen Bedingungen Nukleinsäuren adsorbierendem Material behandelt. Als solches Material wird Diatomeenerde oder andere Siliciumoxid enthaltende mineralische Träger genannt. Es gelingt nach der in der EP 0 389 063 A2 genannten Methode RNA und DNA und RNA und ssRNA gleichzeitig zu isolieren. Eine wünschenswerte Fraktionierung der an dem Siliciumdioxidträger gebundenen Nukleinsäuren in DNA- und RNA-Anteile wird jedoch nicht erreicht. RNA kann dann durch Zugabe von RNAse abgebaut werden, so daß die DNA übrig bleibt.

Gillespie et al. offenbaren im US-Patent-Nr. US 5,155,018 ein Verfahren zur Isolierung und Reinigung von biologisch aktiver RNA aus biologischen Quellen enthaltend RNA, DNA und andere Zellinhaltsstoffe. Dabei wird die RNA enthaltende Quelle mit Partikeln in Kontakt gebracht, welche silicagelhaltige Materialien wie feinverteiltes Glas sind. Der Bindungspuffer, aus welchem die RNA an dem Material adsorbiert wird, ist angesäuerte chaotrope Salze enthaltende Lösungen. Unter diesen Bedingungen wird RNA aber nicht DNA an dem Silicamaterial gebunden. Die Verwendung von angesäuerten chaotropen Puffern besitzt den Nachteil, daß bei der Ansäuerung Guanidiniumthiocyanat (GTC)-haltiger Bindungspuffer die Gefahr der Blausäurebildung besteht und somit besondere Vorsichtsmaßnahmen getroffen werden müssen. Weiterhin wird die DNA durch Säureeinwirkung zerstört. Darüberhinaus läßt sich nach diesem Verfahren eine DNA Aufreinigung aus der authentischen Probe nicht durchführen.

Little beschreibt in US-Patent-Nr. 5,075,430 ein Verfahren zur Reinigung von Plasmid- und anderer DNA, sowohl einzelsträngig wie auch doppelsträngig, durch Immobilisierung der DNA auf Diatomeenerde in Anwesenheit eines chaotropen Agens, woraufhin die DNA mit Wasser oder mit einem Puffer mit niedrigem Salzgehalt eluiert wird. Nach der Methode ist eine Reinigung von DNA/RNA nicht möglich.

M. A. Marko et al. beschreiben in "Analytical Biochemistry" **121**, Seiten 382 bis 387 (1982) ein Verfahren zur Isolierung im großen Maßstab von hochgereinigter Plasmid-DNA unter Verwendung der alkalischen Extraktion und Bindung an Glaspulver. Eine Fraktionierung und getrennte Reinigung von RNA und DNA aus einer einzigen Probe wird nicht beschrieben.

An die Rohpräparation der Nukleinsäuren schließen sich Folgereaktionen an. Diese Folgereaktionen stellen bestimmte Anforderungen sowohl an die Isolierungsprozedur, als auch an die Reinheit und Integrität der isolierten Nukleinsäuren. Insbesondere wenn in der Folge enzymatische Amplifikationsreaktionen wie PCR (Polymerase Chain Reaction), LCR (Ligase Chain Reaction), NASBA (Nucleic Acid Sequence-Based Amplification) oder 3SR (self-sustained Sequence Replication) Anwendung finden, sollte die Präparation der Nukleinsäuren ohne die Gefahr von Kreuzkontaminationen anderer Proben möglich sein, und die isolierten Nukleinsäuren sollten frei von störenden Zellbestandteilen und/oder Metaboliten vorliegen. Die enzymatische Amplifikation von DNA (z. B. PCR) oder RNA (z. B. RNA-PCR) gewinnt aufgrund ihrer Spezifität und Sensitivität nicht nur in der Grundlagenforschung, sondern zunehmend auch im medizinischen Bereich für diagnostische Zwecke an Bedeutung. So beispielsweise für die Detektion von Nukleinsäuresequenzen aus kleinsten Mengen an Zellen und/oder Geweben oder Biopsiematerialien oder zum Nachweis von viralen Nukleinsäuren aus Blut oder Plasma. Für diese Anwendungen sind neben den genannten Anforderungen auch an Ausbeute und Reproduzierbarkeit des Verfahrens zur Nukleinsäureisolierung höchste Anforderungen gestellt.

Ein der Erfindung zugrundeliegendes technisches Problem besteht darin, ein Verfahren anzugeben, mit dem es nicht nur gelingt RNA und DNA aus biologischen Proben wie Zell-Lysaten und GewebeLysaten getrennt voneinander aber aus derselben Probe stammend aufzureinigen, sondern generell doppelsträngige von einzelsträngige Nukleinsäuren. Das Verfahren sollte dabei möglichst kostengünstig arbeiten, indem beispielsweise preisgünstige unmodifizierte Trennmaterialien Verwendung finden können. Das Verfahren sollte darüber hinaus auch zur Probenvorbereitung für die Diagnostik geeignet und mit verschiedenen Amplifikationsmethoden kompatibel sein. Weiterhin sollen die bei der Diskussion des Standes der Technik angesprochenen Nachteile vermieden werden.

Überraschenderweise wird das der Erfindung zugrundeliegende technische Problem durch ein Verfahren mit den Merkmalen des Patentanspruchs 1 in seinen Verfahrensalternativen 1.1 bis 1.4 gelöst. Die Unteransprüche 2 bis 11 betreffen bevorzugte Ausführungsformen des erfindungsgemäßen Verfahrens, die Ansprüche 12 bis 15 die Verwendung wässrigen Lösungen im erfindungsgemäßen Verfahren und Anspruch 16 betrifft eine Zusammenstellung enthaltend die für das erfindungsgemäße Verfahren notwendigen Komponenten.

Im einzelnen stellt sich das erfindungsgemäße Verfahren zur Fraktionierung von doppelsträngigen und einzelsträngigen Nukleinsäure-Strukturen aus biologischen Quellen in folgenden Verfahrensalternativen dar.

Die zu trennende Nukleinsäurearten (einzel- und doppelsträngige) enthaltende Probe wird mit mindestens einem mineralischen Träger behandelt, wobei die Behandlungsbedingungen durch ein entsprechendes wäßriges Gemisch von chaotropen Salzen in einer Konzentration von 1-10 M und niederen aliphatischen Alkoholen mit 1 bis 5 Kohlenstoffatomen in einer Konzentration von 1-90 Vol.-% so eingestellt sind, daß überwiegend die einzelsträngige Nukleinsäure-Fraktion an einem ersten mineralischen Träger adsorbiert wird, während die doppelsträngige Nukleinsäure nicht adsorbiert wird. Die austretende doppelsträngige Nukleinsäure kann dann mit an sich bekannten Verfahren weiter bearbeitet werden. Die am ersten mineralischen Träger adsorbierte einzelsträngige Nukleinsäure wird nach gegebenenfalls durchgeführten Waschschritten eluiert unter Bedingungen geringer Ionenstärke oder mit Wasser. Die nicht adsorbierte doppelsträngige Nukleinsäure, die aufgefangen wurde, kann weiter aufgereinigt werden, indem z.B. die Fraktion anschließend durch ein entsprechendes wäßriges Gemisch von Salzen, insbesondere chaotrope Substanzen, und alkoholgruppenhaltigen Substanzen auf solche Bedingungen eingestellt wird, daß die doppelsträngige Nukleinsäure an einem zweiten mineralischen Träger adsorbierbar und, nach gegebenenfalls durchgeführten Waschschritten, eluierbar wird unter Bedingungen geringer Ionenstärke oder mit Wasser.

In einer zweiten Ausführungsform des erfindungsgemäßen Verfahrens werden zur Trennung von einzelsträngiger Nukleinsäure und doppelsträngiger Nukleinsäure die Behandlungsbedingungen so eingestellt, daß Erdalkali-Ionen komplexierende Substanzen in Abwesenheit von Alkoholgruppen enthaltenden Substanzen in der Lösung enthalten sind, wobei die einzelsträngige Nukleinsäure nicht an dem ersten mineralischen Träger adsorbiert wird und von der übrigen Probe abgetrennt werden kann. Die abgetrennte einzelsträngige Nukleinsäure kann dann mit an sich bekannten Verfahren weiter bearbeitet werden. Die doppelsträngige Nukleinsäure bindet jedoch überwiegend an dem ersten mineralischen Träger und kann, nach gegebenenfalls durchgeführten Waschschritten, eluiert werden, unter Bedingungen geringer Ionenstärke oder mit Wasser. Die so erhaltene doppelsträngige Nukleinsäure kann dann mit an sich bekannten Verfahren weiter bearbeitet werden.

Die nicht adsorbierte einzelsträngige Nukleinsäure, welche aufgefangen wurde, wird dann anschließend durch Zugabe von niederen aliphatischen Alkoholen mit 1 bis 5 Kohlenstoffatomen in einer Kozentration von 1 bis 90 Vol.-% auf solche Bedingungen eingestellt werden, daß die einzelsträngige Nukleinsäure dann an einem zweiten mineralischen Träger adsorbierbar und, nach gegebenenfalls durchgeführten Waschschritten, eluierbar wird unter Bedingungen geringer Ionenstärke oder mit Wasser.

Werden die Behandlungsbedingungen so eingestellt, daß Netz-, Wasch- oder Dispergiermittel in Abwesenheit von Alkoholgruppen aufweisenden Substanzen in der Lösung enthalten sind, wird die einzelsträngige Nukleinsäure unter diesen Behandlungsbedingungen nicht an einem ersten mineralischen Träger adsorbiert und kann mithin von der übrigen Probe abgetrennt und weiter verarbeitet werden. Die doppelsträngige Nukleinsäure bindet jedoch überwiegend an den ersten mineralischen Träger und kann nach gegebenenfalls durchgeführten Waschschritten unter Bedingungen geringer Ionenstärke oder mit Wasser eluiert werden. Die eluierte doppelsträngige Nukleinsäure kann danach mit an sich bekannten Verfahren weiter bearbeitet werden.

Die nicht adsorbierte, aufgefangene einzelsträngige Nukleinsäure wird dann anschließend durch Zugabe von niederen aliphatischen Alkoholen mit 1 bis 5 Kohlenstoffatomen in einer Kozentration von 1 bis 90 Vol.-% auf solche Bedingungen eingestellt werden, daß die einzelsträngige Nukleinsäure dann an einem zweiten mineralischen Träger adsorbierbar und, nach gegebenenfalls durchgeführten Waschschritten, eluierbar wird unter Bedingungen geringer Ionenstärke oder mit Wasser.

Eine weitere Ausführungsform des erfindungsgemäßen Verfahrens gewährleistet die Fraktionierung von gemeinsam gebundener einzelsträngiger Nukleinsäure und doppelsträngiger Nukleinsäure. Dabei werden die Behandlungsbedingungen durch ein entsprechendes wäßriges Gemisch von chaotropen Salzen in einer Konzentration von 1-10 M und niederen aliphatischen Alkoholen mit 1 bis 5 Kohlenstoffatomen in einer Konzentration von 1-90 Vol.-% so eingestellt, daß die Gesamtnukleinsäure aus einzelsträngiger Nukleinsäure und doppelsträngiger Nukleinsäure an einem mineralischen Träger adsorbiert wird, gefolgt von einer Fraktionierung der an den ersten Träger gebundenen doppelsträngigen/einzelsträngigen Nukleinsäure durch selektive Elution der doppelsträngigen Nukleinsäure mittels Behandlung mit einer Lösung verminderter Ionenstärke und Konzentration einer Alkoholgruppen enthaltenden Substanz oder Elution der einzelsträngigen Nukleinsäure mittels einer Lösung enthaltend eine Erdalkali-Ionen komplexierende Substanz und/oder ein Netz-, Wasch- oder Dispergiermittel sowie eine oder mehrere Salzart(en), insbesondere chaotrope Substanzen. Im ersten Fall bleibt dann die einzelsträngige Nukleinsäure auf dem Träger gebunden, wohingegen im zweiten Falle die doppelsträngige Nukleinsäure an dem mineralischen Träger gebunden bleibt. Die jeweils eluierte Fraktion kann dann mit an sich bekannten Verfahren weiter bearbeitet werden.

Die Einstellung der Behandlungsbedingungen mit den Alkoholen und den chaotropen Salzen zur Trennung der Nukleinsäuren erfolgt erfindungsgemäß unter Zugrundelegung der nachstehenden physiko-chemischen Grundlagen, die hier zum ersten Mal formuliert sind.

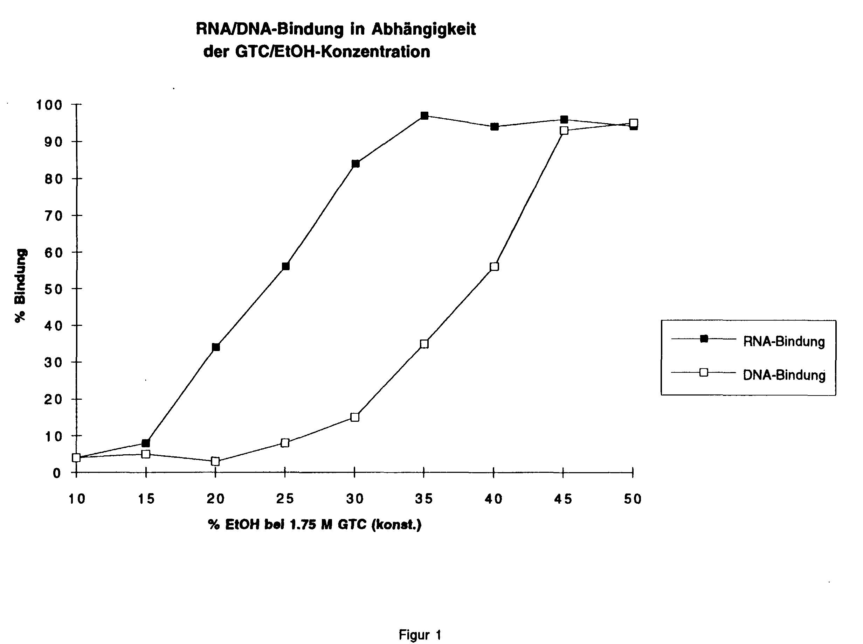

Fig. 1 zeigt die Bindung einzelsträngiger/doppelsträngiger Nukleinsäure am Beispiel einzelsträngiger RNA und doppelsträngiger DNA. Es wird die RNA/DNA-Bindung aus einem Gewebelysat an einen mineralischen Träger in Abhängigkeit der Konzentration einer Alkoholgruppen enthaltenden Substanz (hier Ethanol) und einer chaotropen Substanz (hier GTC) beschrieben. Unter der Voraussetzung, daß die Konzentration einer der Substanzen, Alkohol oder chaotroper Substanz, konstant ist, ist festzustellen, daß bei hoher Alkoholkonzentration und/oder Menge chaotroper Substanz, beide Nukleinsäurearten (RNA/DNA) am mineralischen Träger gebunden werden. Unterschreitet die Konzentration einer oder beider Substanzen (Alkohol oder chaotropes Reagenz) einen bestimmten Wert, dann bindet keine der Nukleinsäure in nennenswertem Maße an dem mineralischen Träger. Dazwischen binden überraschenderweise RNA und DNA so unterschiedlich an den mineralischen Träger, daß dies zur Trennung der Nukleinsäuren ausgenutzt werden kann. So können - ausgehend von Zellen - nach Lyse der Zellen mit hoher Konzentration an chaotropen Substanzen, durch anschließende Zugabe einer Alkoholgruppen enthaltenden Substanz oder eines Gemisches aus Alkoholgruppen enthaltender Substanz und Wasser oder Puffer, Konzentrationen von chaotroper Substanz und Alkoholgruppen enthaltener Substanz so eingestellt werden, daß eine selektive Bindung der RNA erzielt wird, während die DNA im Durchbruch verbleibt. Im Beispiel gemäß Figur 1 würde man eine Konzentration von 1,75 M GTC und 30 Vol% Ethanol wählen, um eine Trennung von RNA von DNA durch fraktionierte Bindung zu erreichen.

Andererseits kann unter Bedingungen hoher Konzentration an Alkoholgruppen enthaltender Substanz und/oder hoher Konzentration an chaotroper Substanz eine simultane Bindung von einzelsträngiger Nukleinsäure und doppelsträngiger Nukleinsäure am mineralischen Träger erreicht werden, und durch Verringerung der Konzentration an Alkoholgruppen enthaltender Substanz und/oder chaotroper Substanz zunächst die Desorption der doppelsträngigen Nukleinsäure eingeleitet werden. Die einzelsträngige Nukleinsäure bleibt dabei gebunden und eluiert bei noch weiterer Verringerung der Konzentration einer oder beider Substanzen. Im Beispiel gemäß Fig. 1 würde man Konzentrationen von 1,75 M GTC und 45 Vol% Ethanol wählen, um eine Bindung der Gesamtnukleinsäure zu erreichen. Wie in Beispiel 8 exemplarisch verdeutlicht wird, würde man zur selektiven Desorption der DNA eine Konzentration von 0,3 M GTC und 10 Vol% Ethanol wählen.

Es ist mithin möglich, die RNA und DNA durch Adsorption an einem mineralischen Träger zu trennen, oder aber zunächst die Gesamtnukleinsäure an den mineralischen Träger zu adsorbieren und die einzelsträngige Nukleinsäure oder doppelsträngige Nukleinsäure selektiv zu eluieren.

Gegebenenfalls können vor der Elution der jeweiligen Nukleinsäure (einzelsträngige Nukleinsäure oder doppelsträngige Nukleinsäure) auch Waschschritte durchgeführt werden.

Die Elution erfolgt dann jeweils unter Bedingungen geringer Ionenstärke oder mit Wasser. Die zuerst vom mineralischen Träger desorbierte Nukleinsäure wird anschließend durch Erhöhung der Ionenstärke und/oder der Konzentration Alkoholgruppen enthaltender Substanzen so eingestellt, daß die doppelsträngige Nukleinsäure oder einzelsträngige Nukleinsäure an einem zweiten mineralischen Träger adsorbiert wird und, nach gegebenenfalls durchgeführten Waschschritten, eluiert wird unter Bedingungen geringer Ionenstärke oder mit Wasser.

Das erfindungsgemäße Verfahren ermöglicht in vorteilhafter Weise die Bearbeitung kleiner Probenmengen, gewährleistet eine einfache und sichere Handhabung unter Vermeidung von Präzipitationsschritten. Weiterhin ist das Verfahren gemäß der Erfindung wenig kosten- und personalintensiv durchführbar und kann in einfacher Weise die Bearbeitung einer Vielzahl von Proben gleichzeitig ermöglichen. Das Verfahren ist aufgrund seiner Vielseitigkeit und einfachen Handhabbarkeit auch für die Automatisierung geeignet.

Erfindungsgemäß können mit dem Verfahren aus Nukleinsäurestrukturen enthaltenden Quellen Doppelstrang/Einzelstrang-Nukleinsäurestrukturen getrennt werden. Als Quellen, die zu trennenden Nukleinsäurestrukturen beinhalten können, kommen die z.B. im Anspruch 7 genannten Quellen in Frage. Diese sind im einzelnen Zellkulturen, Gewebe jeder Art, Körperflüssigkeiten wie Blut, Plasma, Serum, Urin, Faeces, Mikroorganismen wie Bakterien, Viren wie Cytomegalie-Virus, HIV, Hepatitis B, Hepatitis C, Hepatitis-δ-Virus, Pflanzen, Pflanzenteile, Embryonen, Keimlinge, Früchte oder Nukleinsäuren enthaltende Gemische nach enzymatischen Reaktionen wie in vitro Transkription und/oder cDNA-Synthese und/oder reverse Transkription mit anschließender Polymerasekettenreaktion (PCR).

Zellen werden vorzugsweise zunächst in einem wäßrigen Lyse-system, welches chaotrope Substanzen und/oder andere Salze enthält, aufgeschlossen, indem im einfachsten Falle die Zellen damit versetzt werden. Gegebenenfalls kann durch mechanische Einwirkung der Prozeß des Aufschliessens beschleunigt werden.

Danach wird die so behandelte Probe, je nach Problemstellung welche Nukleinsäureart von der anderen getrennt werden soll, wie in den Verfahrensschritten 1.1 bis 1.4 gemäß Patentanspruch 1 beschrieben, weiter aufgearbeitet.

Einige der genannten Ausgangsmaterialien wie beispielsweise Bakterien können, aufgrund der Beschaffenheit ihrer Zellwände, nicht direkt in chaotrope Substanzen enthaltenden wäßrigen Systemen lysiert werden. Diese Ausgangsmaterialien müssen daher, bevor sie in dem erfindungsgemäßen Verfahren eingesetzt werden können, vorbehandelt werden, beispielsweise mit lytischen Enzymen.

Systeme zur Lysierung der die Nukleinsäure enthaltenden Quellen sind vorzugsweise Lösungen chaotroper Substanzen in einer Konzentration von 0,1 bis 10 M. Als chaotrope Substanzen kommen insbesondere Salze wie Natriumperchlorat, Guanidiniumhydrochlorid, Guanidinium-iso-thiocyanat/Guanidinium-thiocyanat, Natriumjodid, Kaliumjodid und/oder Kombinationen davon in Frage.

Auch wäßrige Lösungen enthaltend Salze wie Natriumchlorid, Lithiumchlorid, Kaliumchlorid, Natriumacetat, Magnesiumchlorid in einer Konzentration von 0,1 bis 10 M oder Harnstoff in entsprechenden Konzentrationen von 0,1 bis 10 M und/oder Kombinationen dieser Stoffe können als wäßrige Systeme zur Lyse bzw. Bindung der die Nukleinsäure enthaltenden Quellen verwendet werden.

Die niedere aliphatische Alkohole mit 1 bis 5 Kohlenstoffatomen sind Methanol, Ethanol, Isopropanol, Butanol und Pentanol. Sie werden, in einer Konzentration von 1 bis 90 Vol.-% eingesetzt.

Der mineralische Träger besteht vorzugsweise aus porösen oder nicht porösen Metalloxiden oder Metallmischoxiden, Silicagel, Materialien, die überwiegend aus Glas bestehen, wie nicht modifizierte Glaspartikel, Glasmehl, Quarz, Aluminiumoxid, Zeolithe, Titandioxid, Zirkondioxid mit einer Partikelgröße des mineralischen Trägermaterials von 0,1 µm bis 1.000 µm und einer Porengröße von 2 bis 1.000 µm. Das poröse oder nicht poröse Trägermaterial kann in Form loser Schüttungen vorliegen oder als Filterschichten ausgebildet sein in Form von Filterschichten aus Glas, Quarz oder Keramik und/oder einer Membran, in der Silicagel angeordnet ist und/oder als Partikel oder Fasern aus mineralischen Trägern und Geweben aus Quarz oder Glaswolle vorliegen sowie Latex-Partikeln mit oder ohne funktionellen Gruppen oder Frittenmaterialien aus Polyethylen, Polypropylen, Polyvinyliden-fluorid, insbesondere ultra high molecular weight Polyethylen, high density Polyethylen.

Als Erdalkali-Ionen bindende Substanz kommt insbesondere Ethylendiamintetraessigsäure (EDTA) oder EGTA in Frage und als Netz-, Wasch- oder Dispergiermittel ist ein Sarkosinat einsetzbar.

Gewünschtenfalls können die erfindungsgemäß erhaltenen Nukleinsäuren durch weitere chromatographische Verfahren wie Anionenaustauscherchromatographie gereinigt werden.

Im erfindungsgemäßen Verfahren kann als einzelsträngige Nukleinsäure insbesondere RNA von doppelsträngiger Nukleinsäure (DNA) getrennt werden. Liegt DNA einzelsträngig vor, kann diese DNA dann auch von doppelsträngiger DNA, wie auch von doppelsträngiger RNA getrennt werden.

Die Verwendung im erfindungsgemäßen Verfahren zur Anwendung kommenden Lösungen sind ebenfalls Gegenstand der vorliegenden Erfindung. Als Lysepuffer und/oder Bindungspuffer kommen erfindungsgemäß insbesondere wäßrige Lösungen enthaltend 0,5 bis 8,0 M Guanidinium-iso-thiocyanat/Guanidiniumthiocyanat und/oder Guanidinhydrochlorid, 1 bis 50 % Ethanol und/oder Isopropanol in Betracht.

Als Lösung zum Auswaschen bzw. Eluieren von an dem mineralischen Träger gebundenen Nukleinsäuren kommt eine wäßrige Lösung enthaltend 0,1 bis 3 M Guanidinium-iso-thiocyanat/Guanidiniumthiocyanat und/oder Guanidinhydrochlorid zusammen mit 1 bis 30 Vol. °s Ethanol und/oder Isopropanol in Frage.

Als wäßriges Lösungssystem, das zum Binden von doppelsträngiger Nukleinsäure an mineralische Träger verwendet werden kann, kommt eine wäßrige Lösung enthaltend 1 bis 5 M Guanidin-iso-thiocyanat und/oder 1 bis 8 M Guanidinhydrochlorid mit 0,1 bis 5 % Sarkosinaten oder 5 mM bis 200 mM EDTA in Betracht. Zum Binden von doppelsträngiger Nukleinsäure kommt auch eine Lösung enthaltend 1 bis 5 M Guanidiniumthiocyanat und/oder 1 bis 8 M Guanidiniumhydrochlorid, 5 mM bis 200 mM EDTA oder EGTA in Frage.

Die erfindungsgemäß beanspruchte Zusammenstellung von Komponenten zur Durchführung des Verfahrens in einem Kit weist insbesondere zum Durchfluß geeignete Hohlkörper auf, in dem der oder die mineralische (n) Träger in der bereits weiter oben beschriebenen Form angeordnet ist (sind). Der mineralische Träger kann in loser Schüttung vorliegen, welche zwischen zwei Einrichtungen fixiert ist oder in Form von Membranen, die in dem Hohlkörper angeordnet sind. Desweiteren können in dem Kit Lösungen enthalten sein oder die Bestandteile für die Zusammenstellung der Lösungen in konzentrierter Form. Daraus kann dann der Anwender die jeweils benötigten Lösungen in der notwendigen Konzentration herstellen.

Ein weiterer vorteilhafter Bestandteil der Zusammenstellung ist eine Vorrichtung zur Homogenisierung der Lösung auf Zell-Lysaten. Eine besonders bevorzugte Vorrichtung zur Homogenisierung ist in der internationalen Patentanmeldung WO 95/18851 vorgeschlagen. Diese Vorrichtung besteht im wesentlichen aus mindestens zwei porösen Schichten, wobei die porösen Schichten abnehmende Porengröße aufweisen in Fließrichtung durch die Schichten gesehen. Beim Durchtritt des Zell-Lysats durch die abnehmende Porengrößen werden die viskosen Lösungen des Zell-Lysats homogenisiert.

Die Erfindung wird an den nachfolgenden Beispielen näher erläutert.

### Materialien und Methoden

### 1. Silica-Trägermaterialien

Silica-Trägermaterialien wurden in Membranform oder als suspendierte Partikel eingesetzt.

### 1.1. Silica-Membranen

Zwei Lagen einer Silica-Membran (z.B. Glasfaserfilter der Firma Whatman) wurden, wie in P 43 21 904 beschrieben, in einer Zentrifugen-Chromatographiesäule ("spin-Säule") fixiert. Für membranförmige Trägermaterialien wurde nach dem Standardprotokoll "spin-Prozedur" (vgl. 4.1) vorgegangen.

### 1.2. Silica-Partikel

Verschiedene Silica-Partikel (z.B. der Firmen Merck, Darmstadt und Sigma, St. Louis) wurden als 50 %-Suspension im jeweils verwendeten Lysepuffer (vgl. 3.1) eingesetzt. Die mittleren Partikeldurchmesser betrugen je nach Material 0,5 bis 50 *µ*m. Es wurde nach dem Standardprotokoll "batch-Prozedur" (vgl. 4.2) vorgegangen.

### 2. Nukleinsäure enthaltende Quellen

### 2.1 Gewebe

Die zur Präparation eingesetzten Gewebe wurden sofort nach Entnahme in flüssigem Stickstoff gefroren und bei - 70°C gelagert. Alternativ kann frisches Gewebe verwendet werden.

### 2.2. Pflanzen

Blätter wurden unter flüssigem Stickstoff im Mörser zu einem feinen Pulver zermahlen und direkt zur Präparation eingesetzt oder bei - 70°C gelagert.

### 2.3. Zellkultur

Zellen wurden nach der Ernte zweimal mit PBS gewaschen und pelletiert. Aliquots mit entsprechender Zellzahl (bestimmt durch Auszählen in Thoma-Kammer) wurden frisch zur Präparatiön eingesetzt oder bei - 20°C gelagert.

Adhärent wachsende Zellen können alternativ in der Kulturschale gewaschen und durch Zugabe des jeweiligen Lysepuffers (vgl. 3.1) direkt in der Kulturschale lysiert werden.

### 2.4. Plasma

ACD-Blut wurde 10 min bei 3.000 x g zentrifugiert, der Überstand abgenommen und erneut wie oben zentrifugiert. Der nach der zweiten Zentrifugation erhaltene Überstand wurde aliquotiert und bei - 70°C gelagert.

### 2.5. Bakterien

Kulturen wurden mit einer Übernacht-Kultur angeimpft und bis zu einer OD₆₀₀ von 0,5 - 0,8 angezogen. Aliquots mit der entsprechenden Zellzahl (1 OD₆₀₀ = 10⁹ Zellen/ml) wurden pelletiert und die Zellpellets bei - 20°C gelagert oder frisch zur Präparation eingesetzt.

### 3. Reagenzien

### 3.1. Lysepuffer

- L1: 4,5 M GTC (Guanidiniumthiocyanat), 25 mM NaCitrat pH 7,5, 0,7 % ß-Mercaptoethanol (MSH)
- L2: 4,0 M GTC, 25 mM NaCitrat pH 7,5, 0,7 % ß-MSH
- L3: 5,0 M GTC, 50 mM TRIS/HCl pH 7,0
- L4: 3,5 M GTC, 25 mM NaCitrat pH 7,5, 1 % ß-MSH
- L5: 2,5 M GTC, 25 mM NaCitrat pH 7,5, 1 % ß-MSH, 30 % Ethanol
- L6: 8,0 M GuHCl (Guanidin-Hydrochlorid), 20 mM MOPS pH 7,0, 0,7 % ß-MSH
- L7: 3,0 M GTC, 25 mM NaCitrat pH 7,5, 1 % ß-MSH
- L8: 4,0 M GTC, 50 mM TRIS/HCl pH 7,5, 1 % Sarkosyl
- L9: 4,0 M GTC, 50 mM TRIS/HCl pH 7,5, 25 mM EDTA

### 3.2. Bindungsreagenz

- B1: Ethanol
- B2: n-Butanol
- B3: Isopropanol
- B4: 70 % Ethanol in Wasser
- B5: 5,9 M GTC

### 3.3. Waschpuffer

- W1: 2,0 M GTC, 25 mM TRIS/HCl pH 7,5, 30 % Ethanol
- W2: 4,0 M GTC, 40 mM TRIS/HCl pH 7,5, 20 % Isopropanol
- W3: 1,0 M GTC, 25 mM TRIS/HCl pH 7,5, 20 % Ethanol
- W4: 5,0 M GuHCl, 15 mM MOPS pH 7,0, 37 % Ethanol
- W5: 0,5 M GTC, 25 mM TRIS/HCl pH 7,5, 10 % Ethanol

### 4. Standardprotokolle

### 4.1. "spin-Prozedur"

1) Nukleinsäure enthaltende Quelle mit Lysepuffer (vgl. 3.1.) versetzen und mittels eines Handhomogenisators vollständig homogenisieren
2) Bindungsreagenz (vgl. 3.2.) zugeben, um die jeweiligen Bindebedingungen einzustellen
3) Lysat auf die spin-Säule pipettieren und 15 Sekunden bei 10.000 Upm in einer Tischzentrifuge durch die Membran der spin-Säule zentrifugieren; falls das Volumen des Lysates das Füllvolumen der spin-Säule überschreitet, diesen Bindungsschritt wiederholen
4) den Säulendurchbruch gegebenenfalls weiteraufarbeiten oder verwerfen
5) 700 *µ*l Waschpuffer (vgl. 3.3.) auf die spin-Säule pipettieren und wie in 3) beschrieben, zentrifugieren, um kontaminierende Zellbestandteile zu entfernen
6) die membrangebundenen Nukleinsäuren zweimal mit 700 *µ*l 80 % Ethanol in Wasser salzfrei waschen, hierbei vorgehen wie in 5)
7) die spin-Säule zwei Minuten bei maximaler Drehzahl zentrifugieren, um Ethanol vollständig zu entfernen
8) 50 bis 100 *µ*l auf 80°C erwärmtes Wasser direkt auf die Membran der spin-Säule pipettieren und 1 Minute bei maximaler Drehzahl zentrifugieren, um die Nukleinsäuren zu eluieren; den Elutionsschritt gegebenenfalls wiederholen.

### 4.2. "batch-Prozedur"

1) Nukleinsäure enthaltende Quelle mit Lysepuffer (vgl. 3.1.) versetzen und mittels eines Handhomogenisators vollständig homogenisieren
2) Bindungsreagenz (vgl. 3.2.) zugeben, um die jeweiligen Bindebedingungen einzustellen
3) 50 *µ*l Silica-Suspension (50 % im Lysepuffer) zugeben und zur Bindung der Nukleinsäuren 10 Minuten bei Raumtemperatur inkubieren, dabei mehrmals heftig aufwirbeln (vortexen)
4) 15 Sekunden bei 10.000 Upm in einer Tischzentrifuge zentrifugieren, um das Silica-Material zu pelletieren
5) den Überstand abpipettieren und gegebenenfalls weiteraufarbeiten oder verwerfen
6) 700 *µ*l Waschpuffer (vgl. 3.3.) zum Pellet geben, heftig aufwirbeln (vortexen) bis das Pellet vollständig resuspendiert ist und wie in 4) zentrifugieren
7) Waschschritt 6) zweimal mit 700 *µ*l 80 % Ethanol in Wasser wiederholen, um das Silica-Material salzfrei zu waschen
8) pelletiertes Silica-Material 10 Minuten bei 56°C mit offenem Deckel trocknen
9) 50 bis 100 *µ*l Wasser zugeben, das Pellet durch heftiges Aufwirbeln (Vortexen) komplett resuspendieren und 10 Minuten bei 56°C inkubieren, dabei mehrmals heftig aufwirbeln (vortexen); diesen Elutionsschritt gegebenenfalls wiederholen
10) 1 Minute bei maximaler Drehzahl zentrifugieren und den Überstand in ein neues Reaktionsgefäß überführen.

### 5. Elektrophoretische Methoden

Die isolierten Nukleinsäuren wurden auf Ethidiumbromid-gefärbten Agarosegelen analysiert. Hierzu wurden 1,2 % Formaldehyd- oder 1,2 % 1 x TBE-Gele angefertigt.

Formaldehydgele wurden nach dem Lauf 3 bis 4 Stunden in Wasser, danach über Nacht in 10 *µ*g/ml RNase A geschüttelt, um die RNA zu verdauen und somit die DNA besser sichtbar zu machen. TBE-Gele wurden ohne vorheriges Äquilibrieren RNase-verdaut.

Die im folgenden beschriebenen Beispiele verdeutlichen die Durchführung des erfindungsgemäßen Verfahrens. Alle hiernach isolierten Nukleinsäuren wurden elektrophoretisch analysiert und photometrisch quantifiziert. Der OD 260/280-Wert lag für alle Eluate zwischen 1.7 und 2.0.

### Referenzbeispiele 1 bis 5

### Isolierung von Gesamtnukleinsäure

In den nachfolgend aufgeführten Referenzbeispielen 1 bis 5 wurden die Binde-, Wasch- und Elutionsbedingungen jeweils so gewählt, daß sowohl DNA als auch RNA an den mineralischen Träger binden und gemeinsam eluiert werden.

Es wird anhand dieser Beispiel die Verwendung verschiedener Alkohole (Ethanol, Isopropanol, Butanol) als Bindungsreagenz verdeutlicht.

### Referenzbeispiel 1

### Isolierung von Gesamtnukleinsäure aus Nierengewebe

Aus 15 mg Nierengewebe (Ratte) wurde Gesamtnukleinsäure nach dem Standardprotokoll 4.1 isoliert. Das Gewebe wurde mit 400 *µ*l L1 versetzt und homogenisiert, anschließend wurden 280 *µ*l B1 zugegeben. Der ersten Waschschritt wurde mit W1 durchgeführt, das Elutionsvolumen betrug 2 x 50 *µ*l.

### Referenzbeispiel 2

### Isolierung von Gesamtnukleinsäure aus Lebergewebe

Aus 7 mg Lebergewebe (Ratte) wurde Gesamtnukleinsäure nach dem Standardprotokoll 4.1 isoliert. Das Gewebe wurde mit 300 *µ*l L2 versetzt und homogenisiert, anschließend wurden 200 *µ*l B2 zugegeben. Der erste Waschschritt wurde mit W1 durchgeführt, das Elutionsvolumen betrug 2 x 50 *µ*l.

### Referenzbeispiel 3

### Isolierung von Gesamtnukleinsäure aus HeLa-Zellen

Aus 1 x 10⁶ HeLa-Zellen wurde Gesamtnukleinsäure nach dem Standardprotokoll 4.1 isoliert. Die Zellen wurden mit 400 *µ*l L2 versetzt und homogenisiert, anschließend wurden 200 *µ*l B1 zugegeben. Der erste Waschschritt wurde mit W1 durchgeführt, das Elutionsvolumen betrug 1 x 50 *µ*l.

### Referenzbeispiel 4

### Isolierung von Gesamtnukleinsäure aus Plasma

Gesamtnukleinsäure aus Plasma wurde parallel nach Standardprotokoll 4.1 und 4.2 isoliert. Hierfür wurden jeweils 200 *µ*l Plasma mit 800 *µ*l L3 und 660 *µ*l B2 versetzt und gemischt; homogenisieren war hier nicht erforderlich. Im Ansatz für die "batch-Prozedur" (4.2) wurden zusätzlich 40 *µ*l Silica-Suspension zugegeben. Der erste Waschschritt wurde in beiden Ansätzen mit W2 durchgeführt, das Elutionsvolumen betrug 2 x 100 *µ*l.

### Beispiel 1

### Fraktionierte Bindung von RNA und DNA bei konstanter GTC-Konzentration und steigender Ethanolkonzentration

Die Abhängigkeit der RNA/DNA-Bindung an den mineralischen Träger bei konstanter GTC- und steigender Ethanolkonzentration wurde gezeigt, indem je Probenansatz 10 mg eines Nierengewebes in 350 *µ*l L4 lysiert und zur Einstellung der Ethanolkonzentration je Ansatz 350 *µ*l eines Ethanol/Wasser-Gemisches zugegeben wurde, dessen Ethanolgehalt zwischen 20 und 90 % Ethanol in Wasser lag. Zu einem weiteren Ansatz wurden 350 *µ*l absoluter Ethanol gegeben. Dies entsprach in den jeweiligen Ansätzen Bindebedingungen von konstanter GTC-Konzentration 1,75 M und steigender Ethanolkonzentration im Bereich von 10 bis 50 % (vgl. Fig. 1).

Zu den so eingestellten Lysaten wurden in einer ersten Versuchsreihe je Ansatz 150.000 cpm eines ³²P-markierten 0.9 kb in vitro Transkripts gegeben und das Lysat auf den in eier spin-Säule fixierten mineralischen Träger pipettiert. Es wurde 15 Sekunden bei 10.000 Upm in einer Tischzentrifuge zentrifugiert und die Menge der an die Säule gebundenen und der im Säulendurchbruch befindlichen Radioaktivität durch Cherenkov-Zählung bestimmt.

Die Versuchsreihe wurde wiederholt, wobei statt der ³²P-markierten RNA, 150.000 cpm eines durch Klenow-Auffüllreaktion ³²P-markierten, linearisierten pTZ-Plasmides zugegeben wurden.

Wie Fig. 1 zeigt, bindet die RNA-Fraktion unter den beschriebenen Bedingungen bereits ab Ethanolkonzentrationen größer 25 % an den mineralischen Träger, während die DNA-Fraktion erst ab Ethanolkonzentrationen größer 40 % bindet.

### Beispiele 2 bis 8

### Isolierung von Gesamt-RNA

In den folgenden Beispielen wurden zur Bindung an den mineralischen Träger die Alkohol/Salz-Gemische so gewählt (vgl. Fig. 1), daß eine selektive RNA-Bindung erreicht wurde.

Die Bindebedingungen wurden dabei auf die Art des jeweiligen Aufschlußmaterials (Gewebe, Zellkultur, Pflanzen, Bakterien) abgestimmt.

Die Beispiele verdeutlichen die verwendung von GTC, GuHCl oder GTC/Ethanol-Gemischen zur Lyse der Ausgangsmaterialien. Die Integrität der isolierten RNA wurde durch Northern-blotting oder RT-PCR verifiziert.

Auf die Aufarbeitung der nicht an den Träger gebundenen DNA wurde in diesen Beispiele verzichtet. Die Aufreinigung von DNA aus dem Säulendurchbruch wird in Beispiel 12 gezeigt. Darüber hinaus kann die DNA durch Einstellung der in den Referenzbeispielen 1 bis 5 gewählten Bindebedingungen aufgereinigt werden.

### Beispiel 2

### Isolierung von Gesamt-RNA aus Milzgewebe

Aus 15 mg Milzgewebe (Maus) wurde Gesamt-RNA nach.dem Standardprotokoll 4.1 isoliert. Das Gewebe wurde mit 350 *µ*l L4 versetzt und homogenisiert, anschließend wurden 350 *µ*l B4 zugegeben. Der erste Waschschritt wurde mit W3 durchgeführt, das Elutionsvolumen betrug 1 x 50 *µ*l.

### Beispiel 3

### Isolierung von Gesamt-RNA aus Lebergewebe (A)

In diesem Beispiel wurde ein ethanol-haltiger Lysepuffer verwendet, so daß das Standardprotokoll 4.1 leicht modifiziert wurde.

8 mg Lebergewebe (Ratte) wurden mit 700 *µ*l L5 versetzt und homogenisiert. Das Lysat wurde auf die spin-Säule pipettiert und ab Schritt 3) das Standardprotokoll 4.1 durchgeführt. Der erste Waschschritt wurde mit W3 durchgeführt, das Elutionsvolumen betrug 1 x 50 *µ*l.

### Beispiel 4

### Isolierung von Gesamt-RNA aus Lebergewebe (B)

Aus 15 mg Lebergewebe (Ratte) wurde Gesamt-RNA nach dem Standardprotokoll 4.1 isoliert. Das Gewebe wurde mit 300 *µ*l L6 versetzt und homogenisiert, anschließend wurden 175 *µ*l B1 zugegeben. Der erste Waschschritt wurde mit W4 durchgeführt, das Elutionsvolumen betrug 1 x 50 *µ*l.

### Beispiel 5

### Isolierung von Gesamt-RNA aus HeLa-Zellen

Aus 1 x 10⁷ HeLa-Zellen wurde Gesamt-RNA parallel nach den Standardprotokollen 4.1 und 4.2 isoliert. Die Zellen wurden jeweils mit 350 *µ*l L7 versetzt und homogenisiert, anschließend wurden je 350 *µ* l B4 zugegeben. Im Ansatz für die "batch-Prozedur" (4.2) wurden zusätzlich 50 *µ*l Silica-Suspension zugegeben. Der erste Waschschritt wurde mit W3 durchgeführt, das Elutionsvolumen betrug 1 x 50 *µ*l.

### Beispiel 6

### Isolierung von Gesamt-RNA aus Tabak

Zur Gesamt-RNA-Isolierung aus Pflanzen wird das Standardprotokoll 4.1 leicht modifiziert. Nach Schritt 1) des Protokolls (Lyse), wird ein Zentrifugationsschritt bei 5.000 Upm in einer Tischzentrifuge eingefügt, um nicht lysierte Zellbestandteile, wie z.B. Faserreste, abzutrennen. Der Überstand wird abgenommen, mit Bindungsreagenz versetzt und ab Schritt 2) nach der Standardprozedur weiterverarbeitet.

Aus 100 mg Tabakblättern wurde Gesamt-RNA nach dem für Pflanzen modifizierten Standardprotokoll 4.1 isoliert. Das pulverisierte Zellmaterial wurde mit 600 *µ*l L2 versetzt und homogenisiert, anschließend wurden 350 *µ*l B4 zugegeben. Der erste Waschschritt wurde mit W3 durchgeführt, das Elutionsvolumen betrug 1 x 50 *µ*l.

### Beispiel 7

### Isolierung von GesamC-RNA aus E.coli

Zur Isolierung von Gesamt-RNA aus Bakterien wird vor der Durchführung des Standardprotokolls ein zusätzlicher Arbeitsschritt eingefügt, um die Zellwände der Bakterien zu lysieren. Hierzu wird das Zellpellet in 400 *µ*g/ml Lysozym in TE resuspendiert und 5 min auf Eis sowie 10 min bei Raumtemperatur inkubiert. Anschließend wird nach der Standardprozedur lysiert.

Aus 1 x 10⁹ E.coli wurde Gesamt-RNA nach dem für Bakterien modifizierten Standardprotokoll 4.1 isoliert. Das Pellet wurde in 80 *µ*l 400 *µ*l/ml Lysozym in TE resuspendiert und wie oben beschrieben inkubiert. Anschließend wurden 270 *µ*l L2 zugegeben, homogenisiert und 350 *µ*l B4 zugegeben. Der erste Waschschritt wurde mit W3 durchgeführt, das Elutionsvolumen betrug 2 x 50 *µ*l.

### Beispiel 8

### Selektive RNA-Bindung durch Optimierung des Waschpuffers

Wie dieses Beispiel zeigt, können durch Optimierung des im ersten Waschschrittes verwendeten Waschpuffers (Standardprotokoll 4.1.5) DNA-Kontaminationen von der spezifisch gebundenen RNA entfernt werden.

1 x 10⁶ HeLa-Zellen wurden jeweils nach Standardprotokoll 4.1 in 350 *µ*l L4 lysiert, mit 350 *µ*l B4 versetzt und an den Silica-Träger gebunden. Die Proben wurden dann im ersten Waschschritt mit folgenden Waschpuffer gewaschen:

**Tab. 1**

| Zusammensetzung der Waschpuffer zum Auswaschen von DNA-Kontaminationen | | | |
|---|---|---|---|
| Probe | Waschpuffer | | |
| Nr. | M GTC | 25 mM TRIS/HCl pH 7.5 | % Ethanol |
| 1 | 0.3 | + | 5 |
| 2 | 0.6 | + | 5 |
| 3 | 0.9 | + | 5 |
| 4 | 0.3 | - | 5 |
| 5 | 0.6 | - | 5 |
| 6 | 0.9 | - | 5 |
| 7 - 12 | wie 1-6, jedoch 10 % EtOH | | |
| 8 - 18 | wie 1-6, jedoch 20 % EtOH | | |
| R*) | 1.75 | - | 35 |

| | | | |
|---|---|---|---|
| *) Die Probe diente als Referenz; die Zusammensetzung des Waschpuffers entsprach den Bindebedingungen | | | |

Die weiteren Arbeitsschritte erfolgten nach dem Standardprotokoll; das Elutionsvolumen betrug 1 x 50 µl.

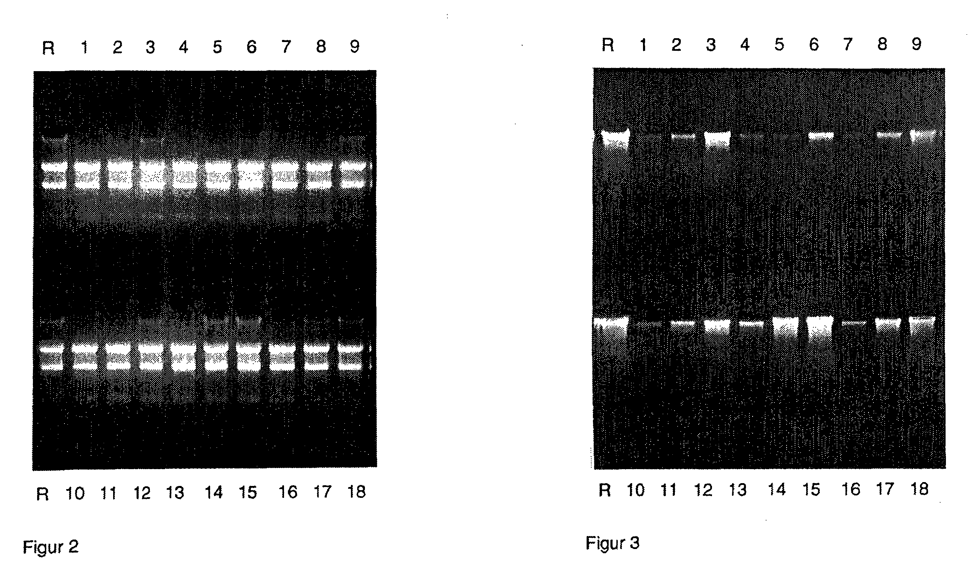

Die Hälfte des Eluates wurde auf einem 1,2 % Formaldehydgel analysiert (vgl. Fig. 2). Das Gel wurde anschließend wie unter "elektrophoretische Methoden" beschrieben mit RNaseA behandelt (vgl. Fig. 3).

### Legende zu Figuren 2 und 3

Fig. 2: 1,2 % Formaldehydgel zur Analyse der Eluate aus Beispiel 8, Bindebedingungen: 1,75 M GTC, 12.5 m;, Nacitrat pH 7.5, 35 % Ethanol; Waschbedingungen: vgl, Tab. 1. Die Benennung der Spuren entspricht der Bezeichnung der Proben in Tabelle 1.

### Fig. 3: RNase-Dau des Geles Fig. 2

### Beispiele 9 und 10

### Isolierung von DNA

In den nachfolgend aufgeführten Beispielen 9 und 10 wurden die Bindebedingungen so gewählt, daß nur die DNA an den mineralischen Träger binden kann, während die RNA durchbricht.

Auf die Aufarbeitung der nicht an den Träger gebundenen RNA wurde in diesen Beispielen verzichtet. Die Aufreinigung von RNA aus dem Säulendurchbruch wird in Beispiel 11 gezeigt. Darüberhinaus kann die RNA im Säulendurchbruch durch Einstellung der in Beispiel 2 bis 8 gewählten Bindebedingungen aufgereinigt werden.

Die selektive DNA-Bindung erfolgt im Lysepuffer in Abwesenheit von Alkohol, d.h. Schritt 2) der Standardprotokolle 4.1 und 4.2 entfällt.

### Beispiel 9

### Isolierung von genomischer DNA aus Nierengewebe

10 mg Nierengewebe (Ratte) wurde in 700 *µ*l L8 lysiert. Die DNA wurde ohne Zugabe von Bindungsreagenz an den mineralischen Träger gebunden und im ersten Waschschritt mit 700 *µ*l L8 gewaschen. Danach wurde das Standardprotokoll 4.1 ab Schritt 6) durchgeführt. Das Elutionsvolumen betrug 2 x 50 *µ*l.

### Beispiel 10

### Isolierung von genomischer DNA aus HeLa-Zellen

1 x 10⁷ HeLa-Zellen wurden in 700 *µ*l L9 lysiert. Die DNA wurde ohne Zugabe von Bindungsreagenz an den mineralischen Träger gebunden und im ersten Waschschritt mit 700 *µ*l L9 gewaschen. Danach wurde das Standardprotokoll 4.1 ab Schritt 6) durchgeführt. Das Elutionsvolumen betrug 2 x 50 *µ*l.

### Beispiele 11 bis 13

### Trennung von Gesamt-RNA und genomischer DNA

Die nachfolgenden Beispiele 11 bis 13 zur getrennten Aufarbeitung von RNA und DNA aus demselben Zell-Lysat stellen Verknüpfungen der oben aufgeführten Beispiele zur RNA-, DNA- bzw. Gesamtnukleinsäureisolierung dar.

Die Trennung kann entweder durch differentielle Bindung oder durch fraktionierte Elution von RNA und DNA erfolgen.

### Beispiele 11 und 12

### Trennung von Gesamt-RNA und genomischer DNA durch differentielle Bindung

Zur differentielle Bindung gibt es wiederum zwei Möglichkeiten:

Nach der Lyse können die Bedingungen entweder so gewählt werden, daß zunächst die DNA an den mineralischen Träger bindet (Beispiel 11), oder aber die RNA kann im ersten Bindungsschritt adsorbiert werden, während die DNA aus dem Durchbruch aufgearbeitet wird (Beispiel 12).

### Beispiel 11

### Isolierung von genomischer DNA und Gesamt-RNA aus Nierengewebe

10 mg Nierengewebe (Ratte) wurden in 350 *µ*l L8 lysiert und die DNA im Lysepuffer an den mineralischen Träger gebunden. Zum Säulendurchbruch wurden 350 *µ*l B4 gegeben und analog Beispiel 3.1 die Gesamt-RNA isoliert. Die Isolierung der genomischen DNA erfolgte wie in Referenzbeispiel 1.

### Beispiel 12

### Isolierung von Gesamt-RNA und genomischer DNA aus Lungengewebe

Aus 20 mg Lungengewebe (Ratte) wurde die Gesamt-RNA wie in Beispiel 2 beschrieben isoliert. Die nicht-gebundene genomische DNA im Säulendurchbruch wurde isoliert, indem 350 *µ*l B1 und 350 *µ*l B5 zugegeben wurden und die DNA wie in Standardprotokoll 4.1 beschreiben an den mineralischen Träger gebunden wurde. Der ersten Waschschritt wurde mit W1 durchgeführt, das Elutionsvolumen betrug 2 x 50 *µ*l.

### Beispiel 13

### Trennung von Gesamt-RNA und genomischer DNA durch fraktionierte Elution

Das folgenden Beispiel verdeutlicht die selektive Elution der DNA-Fraktion der an den mineralischen Träger gebundenen Gesamtnukleinsäure.

Die Bindebedingungen werden so gewählt, daß die Gesamtnukleinsäure an den mineralischen Träger bindet. Die DNA-Fraktion wird anschließend eluiert, während die RNA-Fraktion gebunden bleibt. Die eluierte DNA wird durch erneutes Einstellen auf DNA-Bindebedingungen (vgl. Fig. 1) an einen weiteren mineralischen Träger gebunden und aufgearbeitet.

### Isolierung von genomischer DNA und Gesamt-RNA aus Lebergewebe

15 mg Lebergewebe (Schwein) wurden nach Standardprotokoll 4.1 1) bis 4) in 300 *µ*l L2 lysiert, mit 250 *µ*l B1 versetzt und die Gesamtnukleinsäure an den mineralischen Träger gebunden. Die DNA-Fraktion wurde mit 300 *µ*l W5 eluiert, während das Trägermaterial mit der noch gebundenen RNA-Fraktion ab 5) nach dem Standardprotokoll 4.1 behandelt wurde. Die DNA-Fraktion wurde aus dem Eluat durch Zugabe von 350 *µ*l B1 und 250 *µ*l B5 und Bindung an einen weiteren mineralischen Träger nach dem Standardprotokoll 4.1 isoliert.

## Patentansprüche

1. Verfahren zur Trennung von doppel- und einzelsträngigen Nukleinsäuren aus diese Stoffe enthaltenden Quellen, wobei eine Probe mit mindestens einem mineralischen Träger behandelt wird, wobei
1.1 die Behandlungsbedingungen durch ein wässriges Gemisch von chaotropen Salzen in einer Konzentration von 1 bis 10 M und niederen aliphatischen Alkoholen mit 1 bis 5 Kohlenstoffatomen in einer Konzentration von 1 bis 90 Vol.-% so eingestellt sind, dass überwiegend die einzelsträngige Nukleinsäure enthaltende Fraktion an einem ersten mineralischen Träger adsorbiert wird, während die doppelsträngige Nukleinsäure nicht adsorbiert wird, woraufhin die am ersten mineralischen Träger adsorbierte einzelsträngige Nukleinsäure, nach gegebenenfalls durchgeführten Waschschritten, eluiert wird unter Bedingungen geringer Ionenstärke oder mit Wasser, und für die nicht adsorbierte doppelsträngige Nukleinsäure, die aufgefangen wurde, anschließend durch ein entsprechendes wässriges Gemisch von chaotropen Salzen und in einer Konzentration von 1 bis 10 M und niederen aliphatischen Alkoholen mit 1 bis 5 Kohlenstoffatomen in einer Konzentration von 1 bis 90 Vol.-% solche Bedingungen eingestellt werden, dass diese insbesondere an einem zweiten mineralischen Träger adsorbierbar und, nach gegebenenfalls durchgeführten Waschschritten, vom zweiten mineralischen Träger eluierbar wird unter Bedingungen geringer Ionenstärke oder mit Wasser
oder
1.2 die Behandlungsbedingungen so eingestellt sind, dass Erdalkali-Ionen komplexierende Substanzen in Abwesenheit von niederen aliphatischen Alkoholen mit 1 bis 5 Kohlenstoffatomen in der Lösung enthalten sind, die einzelsträngige Nukleinsäure unter diesen Behandlungsbedingungen nicht an einem ersten mineralischen Träger adsorbiert wird und von der übrigen Probe abgetrennt wird, die doppelsträngige Nukleinsäure jedoch überwiegend an den ersten mineralischen Träger bindet, woraufhin die doppelsträngige Nukleinsäure, nach gegebenenfalls durchgeführten Waschschritten, eluiert wird unter Bedingungen geringer Ionenstärke oder mit Wasser und für die nicht adsorbierte einzelsträngige Nukleinsäure anschließend durch Zugabe von niederen aliphatischen Alkoholen mit 1 bis 5 Kohlenstoffatomen in einer Konzentration von 1 bis 90 Vol.-% Bedingungen eingestellt werden, so dass die einzelsträngige Nukleinsäure dann insbesondere an einen zweiten mineralischen Träger adsorbierbar wird und, nach gegebenenfalls durchgeführten Waschschritten, vom zweiten mineralischen Träge eluierbar wird unter Bedingungen geringer Ionenstärke oder mit Wasser
oder
1.3 die Behandlungsbedingungen so eingestellt sind, dass Netz-, Wasch- oder Dispergiermittel in Abwesenheit von niederen aliphatischen Alkoholen mit 1 bis 5 Kohlenstoffatomen in der Lösung enthalten sind und die einzelsträngige Nukleinsäure unter diesen Behandlungsbedingungen nicht an einem ersten mineralischen Träger adsorbiert wird und von der übrigen Probe abgetrennt wird, die doppelsträngige Nukleinsäure jedoch überwiegend an den ersten mineralischen Träger bindet, woraufhin die doppelsträngige Nukleinsäure, nach gegebenenfalls durchgeführten Waschschritten, vom ersten mineralischen Träger eluiert wird unter Bedingungen geringer Ionenstärke oder mit Wasser und für die nicht adsorbierte aufgefangene einzelsträngige Nukleinsäure anschließend durch Zugabe von niederen aliphatischen Alkoholen mit 1 bis 5 Kohlenstoffatomen in einer Konzentration von 1 bis 90 Vol.-% Bedingungen eingestellt werden, so dass die einzelsträngige Nukleinsäure dann insbesondere an einem zweiten mineralischen Träger adsorbierbar und, nach gegebenenfalls durchgeführten Waschschritten, eluierbar wird unter Bedingungen geringer Ionenstärke oder mit Wasser
oder
1.4 die Behandlungsbedingungen durch ein entsprechendes wässriges Gemisch von chaotropen Salzen in einer Konzentration von 1 bis 10 M und niederen aliphatischen Alkoholen mit 1 bis 5 Kohlenstoffatomen in einer Konzentration von 1 bis 90 Vol.-% so eingestellt sind, dass die Gesamtnukleinsäure (einzel- oder doppelsträngige) an einem ersten mineralischen Träger adsorbiert wird, gefolgt von einer Fraktionierung der an den Träger gebundenen einzel-/doppelsträngigen Nukleinsäuren durch Elution der einzel-/doppelsträngigen Nukleinsäuren vom ersten mineralischen Träger durch selektive Elution der doppelsträngigen Nukleinsäure mittels Behandlung mit einer Lösung verminderter Ionenstärke und Konzentration von niederen aliphatischen Alkoholen mit 1 bis 5 Kohlenstoffatomen oder Elution der einzelsträngigen Nukleinsäure mittels einer Lösung enthaltend eine Erdalkali-Ionen komplexierende Substanz und/oder ein Netz-, Wasch- oder Dispergiermittel sowie eine oder mehrere Salzarten,
wobei die jeweils andere Nukleinsäure an dem ersten mineralischen Träger gebunden bleibt und, nach gegebenenfalls durchgeführten Waschschritten, vom ersten mineralischen Träger eluiert wird unter Bedingungen geringer Ionenstärke oder mit Wasser und für die vorher vom ersten mineralischen Träger eluierte doppel- oder einzelsträngige Nukleinsäure anschließend durch Erhöhung der Ionenstärke oder Konzentration der niederen aliphatischen Alkoholen mit 1 bis 5 Kohlenstoffatomen Behandlungsbedingungen eingestellt werden, die insbesondere zur Adsorption der doppel-/einzelsträngigen Nukleinsäure an einem zweiten mineralischen Träger führen kann und, nach gegebenenfalls durchgeführten Waschschritten, eluierbar wird unter Bedingungen geringer Ionenstärke oder mit Wasser.

2. Verfahren nach Anspruch 1, wobei das System zur Lysierung der die Nukleinsäuren enthaltenden Quellen chaotrope Substanzen in einer Konzentration von 0,1 bis 10 M enthält.

3. Verfahren nach Anspruch 1 und/oder 2, wobei die Nukleinsäuren enthaltende Quelle mit einer Lösung versetzt wird, die Natriumperchlorat, Guanidiniumhydrochlorid, Guanidinium-iso-thiocyanat Guanidinium-thiocyanat, Natriumjodid, Kaliumjodid und/oder Kombinationen davon in einer Konzentration von 0,1 bis 10 M enthält.

4. Verfahren nach mindestens einem der Ansprüche 1 bis 3, wobei die Nukleinsäuren enthaltende Quelle mit einer Lösung versetzt wird, die Natriumchlorid, Lithiumchlorid, Kaliumchlorid, Natriumacetat, Magnesiumchlorid, Harnstoff und/ oder Kombinationen davon in einer Konzentration von 0,1 bis 10 M enthält.

5. Verfahren nach mindestens einem der Ansprüche 1 bis 4, wobei die Alkoholgruppen aufweisenden Substanzen niedere aliphatische Alkohole wie Methanol, Ethanol, Isopropanol, Butanol und Pentanol in einer Konzentration von 1 bis 90 Vol.-% sind und die Salze, wie NaCl, KCl, LiCl, MgCl₂, NaAc, in Konzentration von 1 bis 10 M vorliegen.

6. Verfahren nach mindestens einem der Ansprüche 1 bis 5, wobei der mineralische Träger aus porösen oder nicht porösen Metalloxiden oder Metallmischoxiden, Silicagel, Materialien, die überwiegend aus Glas bestehen, wie nicht modifizierten Glaspartikeln, Glasmehl, Quarz, Aluminiumoxid, Zeolithe, Titandioxid, Zirkondioxid aufgebaut ist mit einer Partikelgröße des mineralischen Trägermaterials von 0,1 im bis 1.000 im und einer Porengröße von 2 bis 1.000 im, und das poröse oder nicht poröse Trägermaterial in Form loser Schüttungen vorliegen kann oder, die Trägermaterialien als Filterschichten ausgebildet sind in Form von Filterschichten aus Glas, Quarz oder Keramik und/oder einer Membran, in der Silicagel angeordnet ist und/oder als Partikel oder Fasern aus mineralischen Trägern und Geweben aus Quarz oder Glaswolle vorliegen sowie Latex-Partikel mit oder ohne funktionellen Gruppen sind oder Frittenmaterialien aus Polyethylen, Polypropylen, Polyvinyliden-fluorid, insbesondere ultra high molecular weight Polyethylen, high density Polyethylen sind.

7. Verfahren nach mindestens einem der Ansprüche 1 bis 6, wobei die biologische Quelle Zellkulturen, Gewebe jeder Art, Körperflüssigkeiten wie Blut, Plasma, Serum, Urin, Faeces, Mikroorganismen wie Bakterien, Viren wie Cytomegalie-Virus, HIV, Hepatitis B, Hepatitis C, Hepatitis-ä-Virus, Pflanzen, Pflanzenteile, Embryonen, Keimlinge, Früchte ist oder die Nukleinsäuren enthaltende Probe Gemische sind, die nach enzymatischen Reaktionen wie in vitro Transkription und/oder cDNA-Synthese und/oder reverse Transkription mit anschließender PCR anfallen.

8. Verfahren nach mindestens einem der Ansprüche 1 bis 7, wobei die Zellen zunächst in einem wässrigen Lyse-System enthaltend chaotrope Substanzen oder andere Salze aufgeschlossen (lysiert) werden oder damit versetzt werden.

9. Verfahren nach mindestens einem der Ansprüche 1 bis 8, wobei jeweils die erhaltenen Fraktionen durch weitere chromatographische Schritte gereinigt werden.

10. Verfahren nach mindestens einem der Ansprüche 1 bis 9, wobei die Erdalkali-Ionen bindende Substanz Ethylendiamintetraessigsäure (EDTA) oder EGTA und das Netz-, Wasch- oder Dispergiermittel ein Sarkosinat ist.

11. Verfahren nach mindestens einem der Ansprüche 1 bis 10, wobei die einzelsträngige Nukleinsäure RNA und die doppelsträngige Nukleinsäure DNA ist.

12. Verwendung einer Lösung enthaltend 0,5 bis 8,0 M Guanidiniumthiocyanat und/oder Guanidiniumhydrochlorid, 1 bis 50% Ethanol und/oder Isopropanol in einem Verfahren nach einem der Ansprüche 1 bis 11.

13. Verwendung einer wässrigen Lösung enthaltend 0,1 bis 3 M Guanidin-thiocyanat und/oder Guanidinhydrochlorid, 1 bis 30% Ethanol und/oder Isopropanol als Puffer zum Auswaschen von an mineralische Träger gebundenen Nukleinsäuren.

14. Verwendung einer wässrigen Lösungen enthaltend 1 bis 5 M Guanidin-thiocyanat und/oder 1 bis 8 M Guanidinhydrochlorid, 0,1 bis 3% Sarkosyl als Puffer zum Binden von DNA an mineralische Träger.

15. Verwendung einer wässrigen Lösung enthaltend 1 bis 5 M Guanidiniumthiocyanat und/oder 1 bis 8 M Guanidiniumhydrochlorid, 5 mM bis 200 mM EDTA oder EGTA als Puffer zum Binden von DNA, doppelsträngiger Nukleinsäure an mineralischen Träger sowie als Puffer um einzelsträngige Nukleinsäure (RNA) nicht an mineralischen Träger zu binden.

16. Kit zur Durchführung des Verfahrens nach mindestens einem der Ansprüche 1 bis 11 mit mineralischem Träger, der in einem zum Durchfluss geeigneten Hohlkörper angeordnet ist, Lösungen, die in den Ansprüchen 12 bis 15 genannt sind sowie weiteren Hilfsstoffen und/oder Zubehör.

## Claims

1. A process for the separation of double-stranded and single-stranded nucleic acids from sources containing such materials wherein a sample is treated with at least one mineral support, wherein
1.1 the treatment conditions are adjusted with an aqueous mixture of chaotropic salts in a concentration of from 1 to 10 M and lower aliphatic alcohols having from 1 to 5 carbon atoms in a concentration of from 1 to 90% by volume in such a way that the fraction containing single-stranded nucleic acid is predominantly adsorbed on a first mineral support whereas the double-stranded nucleic acid is not adsorbed, whereafter, following optionally performed washing steps, said single-stranded nucleic acid adsorbed on said first mineral support is eluted under conditions of low ionic strength or with water, and for the non-adsorbed double-stranded nucleic acid collected, the conditions are subsequently adjusted with an appropriate aqueous mixture of chaotropic salts in a concentration of from 1 to 10 M and lower aliphatic alcohols having from 1 to 5 carbon atoms in a concentration of from 1 to 90% by volume in such a way that said double-stranded nucleic acid becomes adsorbable, in particular, to a second mineral support and, after optionally performed washing steps, becomes elutable from said second mineral support under conditions of low ionic strength or with water; or
1.2 the treatment conditions are adjusted in such a way that materials complexing alkaline earth metal ions are contained in the solution in the absence of lower aliphatic alcohols having from 1 to 5 carbon atoms, the single-stranded nucleic acid is not adsorbed on a first mineral support under these treatment conditions and is separated from the rest of the sample, the double-stranded nucleic acid, however, predominantly binds to said first mineral support, whereafter, following optionally performed washing steps, said double-stranded nucleic acid is eluted under conditions of low ionic strength or with water, and for the non-adsorbed single-stranded nucleic acid, the conditions are subsequently adjusted by the addition of lower aliphatic alcohols having from 1 to 5 carbon atoms in a concentration of from 1 to 90% by volume in such a way that said single-stranded nucleic acid becomes adsorbable, in particular, to a second mineral support and, after optionally performed washing steps, becomes elutable from said second mineral support under conditions of low ionic strength or with water; or
1.3 the treatment conditions are adjusted such that wetting, washing or dispersing agents are contained in the solution in the absence of lower aliphatic alcohols having from 1 to 5 carbon atoms, and the single-stranded nucleic acid is not adsorbed on a first mineral support under such treatment conditions and is separated from the rest of the sample, the double-stranded nucleic acid, however, predominantly binds to said first mineral support, whereafter, following optionally performed washing steps, said double-stranded nucleic acid is eluted from said first mineral support under conditions of low ionic strength or with water, and for the non-adsorbed single-stranded nucleic acid collected, the conditions are subsequently adjusted by addition of lower aliphatic alcohols having from 1 to 5 carbon atoms in a concentration of from 1 to 90% by volume in such a way that said single-stranded nucleic acid becomes adsorbable, in particular, to a second mineral support and, after optionally performed washing steps, becomes elutable under conditions of low ionic strength or with water; or
1.4 the treatment conditions are adjusted with an appropriate aqueous mixture of chaotropic salts in a concentration of from 1 to 10 M and lower aliphatic alcohols having from 1 to 5 carbon atoms in a concentration of from 1 to 90% by volume in such a way that the whole nucleic acid (single-stranded or double-stranded) becomes adsorbed on a first mineral support, followed by fractionation of said single-stranded/double-stranded nucleic acids bound to said support by eluting said single-stranded/double-stranded nucleic acids from said first mineral support by selective elution of said double-stranded nucleic acid by treatment with a solution of reduced ionic strength and reduced concentration of lower aliphatic alcohols having from 1 to 5 carbon atoms, or elution of said single-stranded nucleic acid with a solution containing a material complexing alkaline earth metal ions and/or a wetting, washing or dispersing agent as well as one or more types of salts;
wherein the other nucleic acid, respectively, remains bound to said first mineral support and, after optionally performed washing steps, is eluted from said first mineral support under conditions of low ionic strength or with water, and for the double-stranded or single-stranded nucleic acid first eluted from said first mineral support, treatment conditions are subsequently adjusted by increasing the ionic strength or concentration of lower aliphatic alcohols having from 1 to 5 carbon atoms, which, in particular, may result in adsorption of said double-stranded/single-stranded nucleic acid to a second mineral support, which nucleic acid, after optionally performed washing steps, becomes elutable under conditions of low ionic strength or with water.

2. The process according to claim 1, wherein the system for lysing said sources containing the nucleic acids contains chaotropic substances in concentrations of from 0.1 to 10 M.

3. The process according to claim 1 and/or 2, wherein said source containing the nucleic acids is mixed with a solution containing sodium perchlorate, guanidinium chloride, guanidinium isothiocyanate/guanidinium thiocyanate, sodium iodide, potassium iodide, and/or combinations thereof in concentrations of from 0.1 to 10 M.

4. The process according to at least one of claims 1 to 3, wherein said source containing the nucleic acids is mixed with a solution containing sodium chloride, lithium chloride, potassium chloride, sodium acetate, magnesium chloride, urea, and/or combinations thereof in concentrations of from 0.1 to 10 M.

5. The process according to at least one of claims 1 to 4, wherein said materials containing alcohol groups are lower aliphatic alcohols, such as methanol, ethanol, isopropanol, butanol and pentanol, in concentrations of from 1 to 90% by volume, and said salts, such as NaCl, KCl, LiCl, MgCl₂, NaAc, are present in concentrations of from 1 to 10 M.

6. The process according to at least one of claims 1 to 5, wherein said mineral support consists of porous or non-porous metal oxides or mixed metal oxides, silica gel, materials predominantly consisting of glass, such as unmodified glass particles, powdered glass, quartz, alumina, zeolites, titanium dioxide, zirconium dioxide, the particle size of the mineral support material being from 0.1 µm to 1000 µm and the pore size being from 2 to 1000 µm, and said porous or non-porous support material may be present in the form of loose packings or said support materials are embodied in the form of filter layers made of glass, quartz or ceramics, and/or a membrane in which silica gel is arranged, and/or particles or fibers made of mineral supports and fabrics of quartz or glass wool, as well as latex particles with or without functional groups, or frit materials made of polyethylene, polypropylene, polyvinylidene fluoride, especially ultra high molecular weight polyethylene, high density polyethylene.

7. The process according to at least one of claims 1 to 6, wherein said biological source includes cell cultures, tissues of all kinds, body fluids, such as blood, plasma, serum, urine, faeces; microorganisms, such as bacteria, viruses, such as cytomegaly virus, HIV, hepatitis B, hepatitis C, hepatitis δ virus; plants, plant parts, embryos, germs, fruits, or the samples containing nucleic acids are mixtures obtained from enzymatic reactions, such as in vitro transcription and/or cDNA synthesis and/or reverse transcription with subsequent PCR.

8. The process according to at least one of claims 1 to 7, wherein the cells are first lysed in an aqueous lysis system containing chaotropic substances or other salts, or mixed therewith.

9. The process according to at least one of claims 1 to 8, wherein the fractions obtained are respectively purified by further chromatographic steps.

10. The process according to at least one of claims 1 to 9, wherein said substance binding alkaline earth metal ions is ethylenediaminetetraacetic acid (EDTA) or EGTA, and said wetting, washing or dispersing agent is a sarcosinate.

11. The process according to at least one of claims 1 to 10, wherein said single-stranded nucleic acid is RNA and said double-stranded nucleic acid is DNA.

12. Use of a solution containing from 0.5 to 8.0 M guanidinium thiocyanate and/or guanidinium chloride, and from 1 to 50% ethanol and/or isopropanol in a process according to any of claims 1 to 11.

13. Use of an aqueous solution containing from 0.1 to 3 M guanidinium thiocyanate and/or guanidinium chloride, and from 1 to 30% ethanol and/or isopropanol as a buffer for washing out nucleic acids bound to mineral supports.

14. Use of an aqueous solution containing from 1 to 5 M guanidinium thiocyanate and/or from 1 to 8 M guanidinium chloride, and from 0.1 to 3% sarcosyl as a buffer for binding DNA to mineral supports.

15. Use of an aqueous solution containing from 1 to 5 M guanidinium thiocyanate and/or from 1 to 8 M guanidinium chloride, and from 5 mM to 200 mM EDTA or EGTA as a buffer for binding DNA, double-stranded nucleic acid to mineral supports, and as a buffer for not binding single-stranded nucleic acid (RNA) to mineral supports.

16. A kit for performing the process according to at least one of claims 1 to 11 containing a mineral support which is arranged in a hollow body suited for flow-through, solutions as mentioned in claims 12 to 15, as well as other auxiliaries and/or accessories.

## Revendications

1. Procédé pour séparer des acides nucléiques double brin ou simple brin à partir de sources contenant ces substances, dans lequel un échantillon est traité par au moins un support minéral, où
1.1 les conditions de traitement sont ajustées par un mélange aqueux de sels chaotropiques à une concentration de 1 à 10 M et d'alcools aliphatiques inférieurs de 1 à 5 atomes de carbone à une concentration de 1 à 90 % en volume, de façon qu'essentiellement la fraction contenant l'acide nucléique simple brin soit adsorbée par un premier support minéral, tandis que l'acide nucléique double brin n'est pas adsorbé, ce après quoi l'acide nucléique simple brin adsorbé par le premier support minéral est, après des étapes de lavage éventuellement effectuées, élué dans des conditions d'une faible force ionique ou avec de l'eau, et, pour l'acide nucléique double brin non-adsorbé, qui a été repris, on ajuste ensuite, à l'aide d'un mélange aqueux correspondant de sels chaotropiques et à une concentration de 1 à 10 M, et d'alcools aliphatiques inférieurs ayant de 1 à 5 atomes de carbone à une concentration de 1 à 90 % en volume, des conditions telles que ces derniers puissent être adsorbés en particulier par un deuxième support minéral, et, après des étapes de lavage éventuellement effectuées, puissent être élués du deuxième support minéral dans les conditions d'une faible force ionique, ou avec de l'eau, ou bien
1.2 on ajuste les conditions de traitement de telle sorte que l'on ait dans la solution, en l'absence d'alcools aliphatiques ayant de 1 à 5 atomes de carbone, des substances qui complexent les ions de métaux alcalino-terreux, l'acide nucléique simple brin n'est pas adsorbé par un premier support minéral dans ces conditions de traitement et est séparé du reste de l'échantillon, l'acide nucléique double brin se lie cependant pour l'essentiel au premier support minéral, ce après quoi l'acide nucléique double brin, après des étapes de lavage éventuellement effectuées, est élué dans les conditions d'une faible force ionique ou avec de l'eau, et, pour l'acide nucléique simple brin non-adsorbé, on ajuste ensuite, par addition d'alcools aliphatiques inférieurs ayant de 1 à 5 atomes de carbone à une concentration de 1 à 90 % en volume, des conditions telles que l'acide nucléique simple brin puisse ensuite être adsorbé en particulier par un deuxième support minéral et, après des étapes de lavage éventuellement effectuées, puisse être élué du deuxième support minéral dans les conditions d'une faible force ionique ou avec de l'eau, ou bien
1.3 les conditions de traitement sont ajustées de façon que la solution contienne des mouillants, des agents lavants ou des dispersants, en l'absence d'alcools aliphatiques inférieurs ayant de 1 à 5 atomes de carbone, et l'acide nucléique simple brin, dans ces conditions de traitement, n'est pas adsorbé par un premier support minéral et est séparé du reste de l'échantillon, l'acide nucléique double brin se lie cependant pour l'essentiel au premier support minéral, ce après quoi l'acide nucléique double brin, après des étapes de lavage éventuellement effectuées, est élué du premier support minéral dans les conditions d'une faible force ionique ou avec de l'eau, et, pour l'acide nucléique simple brin repris et non-adsorbé, on ajuste ensuite, par addition d'alcools aliphatiques inférieurs ayant de 1 à 5 atomes de carbone à une concentration de 1 à 90 % en volume, des conditions telles que l'acide nucléique simple brin puisse ensuite être adsorbé en particulier par un deuxième support minéral et, après des étapes de lavage éventuellement effectuées, puisse être élué dans les conditions d'une faible force ionique ou avec de l'eau, ou bien
1.4 les conditions de traitement sont ajustées par un mélange aqueux correspondant de sels chaotropiques à une concentration de 1 à 10 M et d'alcools aliphatiques inférieurs ayant de 1 à 5 atomes de carbone à une concentration de 1 à 90 % en volume, de telle sorte que l'acide nucléique total (simple brin et double brin) soit adsorbé par un premier support minéral, ce après quoi on procède à un fractionnement des acides nucléiques simple brin/double brin liés au support par élution des acides nucléiques simple brin/double brin à partir du premier support minéral, par élution sélective de l'acide nucléique double brin grâce à un traitement par une solution ayant une force ionique réduite et une concentration réduite d'alcools aliphatiques inférieurs ayant de 1 à 5 atomes de carbone, ou par élution de l'acide nucléique simple brin à l'aide d'une substance qui complexe les ions de métaux alcalino-terreux et/ou un mouillant, agent lavant ou dispersant, ainsi qu'une ou plusieurs espèces de sels,
où l'autre acide nucléique reste lié au premier support minéral et, après des étapes de lavage éventuellement effectuées, est élué du premier support minéral dans les conditions d'une faible force ionique ou avec de l'eau, et, pour l'acide nucléique double brin ou simple brin qui a été élué au préalable du premier support minéral, on ajuste ensuite, par augmentation de la force ionique ou concentration des alcools aliphatiques inférieurs ayant de 1 à 5 atomes de carbone, des conditions de traitement qui peuvent conduire en particulier à l'adsorption de l'acide nucléique double brin/simple brin par un deuxième support minéral, et, après des étapes de lavage éventuellement effectuées, peut être élué dans les conditions d'une faible force ionique ou avec de l'eau.

2. Procédé selon la revendication 1, dans lequel le système destiné à la lyse des sources contenant les acides nucléiques contient des substances chaotropiques à une concentration de 0,1 à 10 M.

3. Procédé selon la revendication 1 et/ou 2, dans lequel la source contenant des acides nucléiques est additionnée d'une solution qui contient du perchlorate de sodium, du chlorhydrate de guanidinium, de l'isothiocyanate de guanidinium, du thiocyanate de guanidinium, de l'iodure de sodium, de l'iodure de potassium et/ou une combinaison de ceux-ci à une concentration de 0,1 à 10 M.

4. Procédé selon au moins l'une des revendications 1 à 3, dans lequel la source contenant des acides nucléiques est additionnée d'une solution qui contient du chlorure de sodium, du chlorure de lithium, du chlorure de potassium, de l'acétate de sodium, du chlorure de magnésium, de l'urée et/ou des combinaisons de ceux-ci, à une concentration de 0,1 à 10 M.

5. Procédé selon au moins l'une des revendications 1 à 4, dans lequel les substances comportant des groupes alcools sont des alcools aliphatiques inférieurs tels que le méthanol, l'éthanol, l'isopropanol, le butanol et le pentanol, à une concentration de 1 à 90 % en volume, et les sels, tels que NaCl, KCl, LiCl, MgCl₂, NaAc, sont présents à une concentration de 1 à 10 M.

6. Procédé selon au moins l'une des revendications 1 à 5, dans lequel le support minéral est constitué d'oxydes métalliques poreux ou non-poreux ou d'oxydes métalliques mixtes, de gel de silice, de matériaux qui pour l'essentiel sont constitués de verre, tels que les particules de verre non-modifiées, le verre pulvérisé, le quartz, l'oxyde d'aluminium, les zéolites, le dioxyde de titane, le dioxyde de zirconium, avec une granulométrie du matériau support minéral de 0,1 à 1000 im et un diamètre des pores de 2 à 1000 im et le matériau support poreux ou non-poreux peut se présenter sous forme de masses en vrac, ou encore les matériaux supports sont constitués sous forme de couches filtrantes, sous forme de couches filtrantes en verre, quartz ou céramique et/ou sous forme d'une membrane dans laquelle est disposé du gel de silice, et/ou se présentent sous forme de particules ou de fibres constituées de supports minéraux, et de tissus de quartz ou de laine de verre, et sont des particules de latex avec ou sans groupes fonctionnels, ou encore sont des matériaux frittés à base de polyéthylène, de polypropylène, de poly(fluorure de vinylidène), en particulier de polyéthylène à très grande masse moléculaire, de polyéthylène haute densité.

7. Procédé selon au moins l'une des revendications 1 à 6, dans lequel la source biologique est constituée de cultures cellulaires, de tissus de tous types, de fluides corporels tels que le sang, le plasma, le sérum, l'urine, les fèces, de microorganismes tels que les bactéries, de virus tels que les cétomégalovirus, le VIH, le virus de l'hépatite B, le virus de l'hépatite C, le virus de l'hépatite ä, de plantes, de parties de plantes, d'embryons, de plantules, de fruits, ou encore les échantillons contenant des acides nucléiques sont des mélanges qui sont obtenus après des réactions enzymatiques, telles qu'une transcription in vitro et/ou une synthèse d'ADNc et/ou une transcription inverse suivie d'une PCR.

8. Procédé selon au moins l'une des revendications 1 à 7, dans lequel les cellules sont d'abord attaquées (lysées) dans un système de lyse aqueux contenant des substances chaotropiques ou d'autres sels, ou sont mélangées à ces derniers.

9. Procédé selon au moins l'une des revendications 1 à 8, dans lequel les fractions obtenues sont purifiées par des étapes chromatographiques supplémentaires.

10. Procédé selon au moins l'une des revendications 1 à 9, dans lequel la substance liant les ions de métaux alcalino-terreux est l'acide éthylènediaminetétraacétique (EDTA) ou l'EGTA, et le mouillant, agent lavant ou dispersant est un sarcosinate.

11. Procédé selon au moins l'une des revendications 1 à 10, dans lequel l'acide nucléique simple brin est un ARN, et l'acide nucléique double brin est un ADN.

12. Utilisation d'une solution contenant de 0,5 à 8,0 M de thiocyanate de guanidinium et/ou de chlorhydrate de guanidinium, de 1 à 50 % d'éthanol et/ou d'isopropanol, dans un procédé selon l'une des revendications 1 à 11.

13. Utilisation d'une solution aqueuse contenant de 0,1 à 3 M de thiocyanate de guanidinium et/ou de chlorhydrate de guanidinium, de 1 à 30 % d'éthanol et/ou d'isopropanol, en tant que tampon pour laver des acides nucléiques liés à des supports minéraux.

14. Utilisation d'une solution aqueuse contenant de 1 à 5 M de thiocyanate de guanidinium et/ou de 1 à 8 M de chlorhydrate de guanidinium, de 0,1 à 3 % de sarcosil servant de tampon pour lier l'ADN à des supports minéraux.

15. Utilisation d'une solution aqueuse contenant de 1 à 5 M de thiocyanate de guanidinium et/ou de 1 à 8 M de chlorhydrate de guanidinium, de 5 mM à 200 mM d'EDTA ou d'EGTA, en tant que tampon pour lier l'ADN, l'acide nucléique double brin, à un support minéral, et aussi en tant que tampon pour ne pas lier un acide nucléique simple brin (ARN) à un support minéral.

16. Kit pour mettre en oeuvre le procédé selon au moins l'une des revendications 1 à 11, comportant un support minéral qui est disposé dans un corps creux permettant le passage, des solutions telles que mentionnées dans les revendications 12 à 15, ainsi que d'autres adjuvants et/ou accessoires.
